Europäisches Patentamt

European Patent Office

Office européen des brevets

(11) Publication number: **0 295 622 B1**

# EUROPEAN PATENT SPECIFICATION

(45) Date of publication of patent specification: **29.09.93**  (51) Int. Cl.5: **C12P 13/22**, C12N 9/88

(21) Application number: **88109445.2**

(22) Date of filing: **14.06.88**

The file contains technical information submitted after the application was filed and not included in this specification

(54) **Process for producing L-Tryptophan.**

(30) Priority: **15.06.87 JP 146972/87**
**22.07.87 JP 181237/87**
**16.11.87 JP 287525/87**

(43) Date of publication of application:
**21.12.88 Bulletin 88/51**

(45) Publication of the grant of the patent:
**29.09.93 Bulletin 93/39**

(84) Designated Contracting States:
**BE DE FR GB NL**

(56) References cited:
EP-A- 0 099 742
US-A- 3 808 101

A. Lehninger, BIOCHEMISTRY, 1975; pp.
335-342&NUM;

(73) Proprietor: **RESEARCH ASSOCIATION FOR UTILIZATION OF LIGHT OIL**
**4-11, Shiba 3-chome Minato-ku**
**Tokyo(JP)**

(72) Inventor: **Yamagata, Hisashi**
**2-15-19-401, Toride**
**Toride-shi Ibaraki-ken(JP)**
Inventor: **Endo, Fuzio**
**5220, Ami Ami-machi**
**Inashiki-gun Ibaraki-ken(JP)**
Inventor: **Shimazu, Mitsunobu**
**8-3-12, Chuo Ami-machi**
**Inashiki-gun Ibaraki-ken(JP)**
Inventor: **Terasawa, Masato**
**1-11-5-401, Chuo Ami-machi**
**Inashiki-gun Ibaraki-ken(JP)**
Inventor: **Yukawa, Hideaki**
**6-23-9, Chuo Ami-machi**
**Inashiki-gun Ibaraki-ken(JP)**

(74) Representative: **Kraus, Walter, Dr. et al**
**Patentanwälte Kraus, Weisert & Partner**
**Thomas-Wimmer-Ring 15**
**D-80539 München (DE)**

**Description**

This invention relates to a novel process for producing L-tryptophan. More specifically, it relates to a process for producing optically active L-tryptophan or 5-hydroxytryptophan from fumaric acid in one pot using enzymes.

L-tryptophan is an essential amino acid and is important both nutritionally and physiologically. Since its production in large quantities is now difficult, its use is limited mainly to pharmaceuticals. If a technique of producing large quantities of L-tryptophan at low cost is established, L-tryptophan will find new applications, for example as foods, feed additives and polymeric materials.

L-5-hydroxytryptophan is an amino acid whose unique physiological activity attracts attention. So far, no effective method has been established for producing this amino acid, and it is desired to develop an industrial process for producing L-5-hydroxytryptophan at low cost.

Conventional methods for producing these two amino acids include, for example, a method involving the use of indole or 5-hydroxyindole, an ammonium ion, and pyruvic, oxaloacetic or L-malic acid (for example, U.S. Patent No. 3,808,101). This method, however, is unsuitable for industrial practice because the pyruvic acid, oxaloacetic acid and L-malic acid are very expensive.

Previously, we developed a process for efficiently producing L-tryptophan or L-5-hydroxytryptophan by using fumaric acid which is industrially available at low cost, as a starting material, and reacting it with an ammonium ion and indole or 5-oxyindole in the presence of (A) fumarase, (B) pyruvic acid-malic acid carboxylase and (C) tryptophanase, and applied for patent thereon (see Japanese Laid-Open Patent Publication No.105,687/1988).

EP-A-0 099 742 describes a biochemical enzymatic reaction involving an oxidizing enzyme which converts a substrate to an oxidized product with $NAD^+$ or $NADP^+$ as coenzyme is provided with a recycling system for returning the NADH or NADPH produced to the oxidized state by the presence of a NADH or NADPH oxidase.

We have further extensively studied the enzyme reaction conditions of this process in order to produce L-tryptophan or L-5-hydroxytryptophan more efficiently in higher yields, and have now found that if NADH oxidase is caused to be present in the reaction system, L-tryptophan or L-5-hydroxytryptophan can be produced more efficiently.

Thus, the present invention provides a process for producing L-tryptophan optionally substituted at the 5-position by a hydroxyl group, which comprises enzymatically reacting fumaric acid or its salt, an ammonium ion and indole optionally substituted at the 5-position by a hydroxyl group in the presence of (A) fumarase, (B) pyruvic acid-malic acid carboxylase, (C) tryptophanase and (D) NADH oxidase, wherein (D) NADH oxidase is added to the reaction system in an amount sufficient to increase the yield of said L-tryptophan optionally substituted at the 5-position by a hydroxyl group.

The process of this invention consists of (i) a reaction of forming L-malic acid from fumaric acid by the action of fumarase, (ii) a reaction of converting the resulting L-malic acid into pyruvic acid by the action of pyruvic acid-malic acid carboxylase (denoting an enzyme having the ability to convert L-malic acid into pyruvic acid), (iii) a reaction of the pyruvic acid with an ammonium ion and indole optionally substituted by a hydroxyl group at the 5-position (to be referred to as the indole compound) under the action of tryptophanase to give the desired L-tryptophan optionally substituted by a hydroxyl group at the 5-position, and (iv) a reaction of converting reduced-type coenzyme NADH formed by the reaction (II) into oxidized-type coenzyme $NAD^+$ by NADH oxidase. These reactions are carried out in one pot. Sources of supply of the enzymes (A) to (D) used in the enzyme reactions (i) to (iv) are not particularly limited so long as they have catalytic actions in these reactions. They may be derived from microorganisms, animals or plants. The enzymes used need not to be in an isolated pure form. For example, microorganism cells containing the enzyme, or a treated product thereof may be used. Microorganism cells containing a plurality of enzymes, or a treated product thereof may also be used conveniently.

Examples of the treated product of microorganism cells include the microorganism cells immobilized to a polyacrylamide gel, a carrageenan gel or a polymeric membrane; a lyophilized product of the microorganism cells; a disrupted product of the microorganism cells obtained by ultrasonic oscillation; a mechanically crushed product of the microorganism cells obtained by using a ball mill or the like; and products of treatment of the microorganism cells with solvents or surface-active agents.

The above microorganism cells or the treated products thereof may contain only one of fumarase (A), pyruvic acid-malic acid carboxylase (B), tryptophanase (C) and NADH oxidase (D), or two or more of them at the same time. In the latter case, the microorganism cells or the treated products thereof may be used commonly in two or more of the reactions (i) to (iv).

Microorganisms of the genus Brevibacterium may be cited as examples of microorganisms having the ability to produce fumarase (A) and pyruvic acid-malic acid carboxylase (B) simultaneously. Specific examples are Brevibacterium flavum MJ-233 (FERM BP-1497) and Brevibacterium flavum MJ-233-AB-41 (FERM BP-1498). These microorganism strains may be used commonly in the above reactions (i) and (ii).

Some microorganims of the genus Escherichia, Proteus or Erwinia have the ability to produce fumarase (A), pyruvic acid-malic acid carboxylase (B) and tryptophanase (C) at the same time. Specific examples include Escherichia coli K-12 strain (ATCC 27325), Escherichia coli K-12 strains [YK3002 (FERM BP-1733) and YK3003 (FERM BP-1734)], Proteus morganii (IFO-3848), and Erwinia herbicola (ATCC 21433). These microorganism strains may be used commonly in the reactions (i), (ii) and (iii).

Cells of microorganisms belonging to the genus Brevibacterium are preferred as a source of supply of fumarase (A) and pyruvic acid-malic acid carboxylase (B) because they show a very little decrease in enzyme activity and can be re-used.

It has been found in accordance with this invention that when the same microorganism is used as a source of supply of at least the tryptophanase (C) and pyruvic acid-malic acid carboxylase (B) and cultivated in a medium containing malic acid or its salt as a carbon source, the pyruvic acid-malic acid carboxylase activity of this microorganism can be induced and enhanced substantially without adversely affecting its tryptophanase activity, and microorganism cells having enhanced activities of producing the two enzymes can be obtained, and that by carrying out the above reaction in the presence of the resulting microorganism cells or the treated product thereof, the desired L-tryptophan or its 5-hydroxyl-substituted derivative can be obtained in increased yields.

Such a microorganism which has the ability to produce at least pyruvic acid-malic acid carboxylase (B) and tryptophanase (C) simultaneously and can be cultivated using malic acid or its salt as a carbon source may be, for example, a tryptophanase-producing microorganism of the species Escherichia coli transformed with a plasmid at least containing a structure gene of tryptophanase. Specific examples are Escherichia coli K-12 YK3002 (FERM BP-1733), Escherichia coli K-12 YK3003 (FERM BP-1734), and Escherichia coli K-12 YK3004 (FERM BP-1735).

A source of supply of NADH oxidase (D) may be a microorganism, an animal or a plant. For example, it may be Leuconostoc mesenteroides (ATCC 9135) and Bacillus cereus (ATCC 4342).

Among these microorganisms described above, Brevibacterium flavum MJ-233 (FERM BP-1497), Brevibacterium flavum MJ-233-AB-41 (FERM BP-1498), Escherichia coli YK3002 (FERM BP-1733), Escherichia coli YK3003 (FERM BP-1734) and Escherichia coli YK3004 (FERM BP-1735) have been deposited under deposit numbers FERM BP-1497, FERM BP-1498, FERM BP-1733. FERM BP-1734 and FERM BP-1735, respectively, at Fermentation Research Institute, Agency of Industrial Science and Technology, 1-3, Higashi 1 chome, Tsukuba-shi, Ibarakiken 305, Japan under the Budapest Treaty. Escherichia coli (ATCC 27325), Erwinia herbicola (ATCC 21433), Leuconostoc mesenteroides (ATCC 9135) and Bacillus cereus - (ATCC 4342) are microorganisms described in American Type Culture Collection, Catalogue of Bacteria and Phages. Proteus morganii (IFO 3848) has been deposited at Institute for Fermentation Osaka, 17-85, Juso-honmachi 2-chome, Yodogawa-Ku, Osaka 532, Japan. These microorganisms are freely available to the public.

The enzymes used in the process of this invention may be in the form of microorganism cells or a treated product thereof. It may also be a commercial enzyme preparation. Examples of the enzyme preparation are EC4.2.1.2 which is fumarase (A), EC 1.1.1.38 and EC 1.1.1.39 which are pyruvic acid-malic acid carboxylase (B) and EC4.1.99.1 which is tryptophanase (C).

As required, the enzymes or microorganisms containing the enzymes or the treated product thereof may be used in a form immobilized to a suitable carrier. Immobilization may be carried out by a standard technique described in textbooks on enzymes [for example, "Methods in Enzymology", vol. 44 (1977), editor K. Mosbach, vol. 135 (1987), editor K. Mosbach, vol. 136 (1987), editor K. Mosbach: Academic Press].

The process of this invention can be carried out in the same way as in an ordinary enzyme reaction. For example, fumaric acid or its salt, an ammonium ion source and the indole compound as substrates are fed into a reactor, and the process is carried out in water or an aqueous solution of a buffer such as 0.1M phosphate buffer at a pH of generally 6 to 9, preferably 7 to 8.5, and a temperature of about 20 to 50 °C, preferably about 30 to 40 °C The reaction time is generally about 1 to 72 hours. The concentrations of fumaric acid or its salt and ammonium ion in the reaction system are not strictly limited. The suitable concentration of each of these is generally 0.1 to 20 % (wt/vol), preferably 0.2 to 18% (wt/vol). The concentration of the indole compound is neither restricted strictly. Conveniently, it is generally 0.1 to 20 % (wt/vol), preferably 0.2 to 10% (wt/vol). Desirably, the indole compound is added continuously or intermittently under such controlled conditions that the average concentration of the indole compound in the reaction system does not exceed 4 mM, preferably 3 mM. This makes it possible to avoid the additive inhibiting tendency of tryptophanase with the passage of the reaction, and the yield of L-tryptophan can be increased.

The term "average concentration of the indole compound", as used herein, denotes the concentration of the indole compound which is present in the reaction mixture at any given time during performance of the reaction.

The average concentration of the indole compound in the reaction system may be controlled by, for example, continuously or intermittently (consecutively) measuring and monitoring the concentration of the indole compound in the reaction mixture by colorimetry in accordance with a customary method [e.g., O. H. Smith and C. Yanofsky, "Methods in Enzymology", Academic, New York, (1962), vol. 5, pages 794-806], and adding the indole compound so that its concentration does not exceed 4 mM.

As required and desirably, oxidized-type nicotinamideadenine dinucleotide ($NAD^+$) or reduced-type nicotinamideadenine dinucleotide (NADH), and/or a metal ion may be introducd into the reaction system.

Considerable amounts of the oxidized-type or reduced-type nicotinamideadenine dinucleotide ($NAD^+$ or NADH) and the metal ion are contained in the microoganism cells used as the source of the enzymes (A) to (D). If, therefore, the microorganism ceils are directly used as the enzyme source, required amounts of $NAD^+$ or NADH and/or the metal ion may be secured in the reaction system without separately adding them. Generally, however, it is advantageous to add $NAD^+$ or NADH and/or the metal ion to the reaction system.

The concentration of $NAD^+$ or NADH to be present in the reaction system is not critical, but its suitable concentration is generally 0.005 to 20 mM, preferably 0.01 to 10 mM. Accordingly, when the microorganism cells are used as the enzyme source but the concentration of NAD or NADH in the reaction system does not fall within the above range or when the treated microorganism cells or the enzyme preparation is used, it is desirable to add $NAD^+$ or NADH so that the total concentration of $NAD^+$ or NADH in the reaction system reaches a value within the above range.

Examples of the metal ion which can be introduced into the reaction system are $Mn^{2+}$, $Mg^{2+}$, $Co^{2+}$, $Ni^{2+}$ and $Zn^{2+}$. $Mn^{2+}$, $Mg^{2+}$ and $Co^{2+}$ are preferred. The concentration of the metal ion in the reaction system is neither critical. Its suitable concentration is generally 0.1 to 20 mM, preferably 0.1 to 15 mM. The metal ion may be added usually in the form of a water-soluble salt such as a chloride or sulfate to the reaction system. If a sufficient amount of the metal ion is secured in the reaction system as a result of using the microorganism cells as the enzyme source, it is not necessary to add the metal ion separately to the reaction system. Generally, however, it is preferred to add the metal ion to the reaction system.

The sequence of adding fumaric acid or its salts, the ammonium ion source and the indole compound as reaction substrates is not particularly limited, and they may be added in any desired sequence.

When fumaric acid is used as a free acid, it is normally used after it is neutralized to the aforesaid pH with an alkali such as sodium hydroxide, potassium hydroxide or aqueous ammonia. Since, however, the process of this invention involves an ammonium ion as one reaction component, aqueous ammonia is used preferably for the neutralization. The salt of fumaric acid is not particularly restricted and its ammonium, sodium, calcium and potassium salts may be used. Fumaric acid or its salt may be added at the start in the aforesaid concentration. Alternatively, it may be added consecutively to give a final concentration within the aforesaid range.

Examples of suitable sources of the ammonium ion are ammonia, ammonium sulfate, ammonium chloride, ammonium phosphate and ammonium acetate. These ammonium ion sources may be added at the start in the concentrations described above. Or they may be added consecutively to give a final concentration within the aforesaid range.

According to another aspect of this invention, there is provided a process in which the aforesaid reaction is carried out in the presence of a reducing agent. The presence of the reducing agent prevents the decrease of the activities of the enzymes (A) to (D) caused by oxidation, and therefore, can maintain the stability of the enzymes.

The type and concentration of the reducing agent used in this embodiment are not particularly restricted unless they substantially adversely affect the activities of the enzymes (A) to (D). Generally, it is convenient to use dithiothreitol, dithioerythritol, 2-mercaptoethanol, L-cysteine, L-ascorbic acid or sodium sulfite for example, as the reducing agent in a concentration of 0.0001 to 10 % (wt/vol), preferably 0.001 to 2 % (wt/vol).

The amounts of the enzyme or the enzyme-containing microorganism cells or the treated product thereof are neither strictly limited, and can be varied widely according to the enzyme activity, etc. Suitably, fumarase (A), pyruvic acid-malic acid carboxylase (B) and tryptophanase (C) are used each in a concentration of generally 0.1 to 10 % (wt/vol), preferably 0.1 to 8 % (wt/vol). Advantageously, NADH oxidase (D) is used in a concentration of generally 0.1 to 50 % (wt/vol), preferably 0.1 to 10 % (wt/vol).

The microroganism strain that can be used as a source of supply or the enzyme in the process of this invention may be cultivated in a synthetic or natural medium in accordance with a method known per se. Carbon sources for such a medium include carbohydrates such as glucose, glycerol, fructose, sucrose and molasses for microorganisms of the genera Escherichia, Proteus and Erwinia; and ethanol in addition to the above carbohydrates for microorganisms of the genus Brevibacterium. Examples of nitrogen sources are natural organic nitrogen sources such as tryptone, yeast extract, corn steep liquor and a hydrolyzate of casein. These nitrogen sources may concurrently serve as carbon sources. Other nutrient sources that may be added include minerals such as phosphates (e.g., monopotassium phosphate or dipotassium phosphate), iron sulfate, iron chloride, manganese sulfate, manganese chloride, magnesium sulfate and magnesium chloride. As required, vitamins such as biotin and thiamine hydrochloride may also be added as the nutrient sources.

When a microorganism having the ability to produce at least pyruvic acid-malic acid carboxylase (B) and tryptophanase (C) simultaneously is to be cultivated using malic acid or its salt as a main carbon source, L-or DL-malic acid is added to the culture medium in a concentration of 0.1 to 30 % (wt/vol), preferably 0.5 to 20 % (wt/vol). This concentration may be the initial concentration or the final concentration attained as a result of consecutive addition. The salt of malic acid may be, for example, a sodium, potassium or ammonium salt.

The cultivation can be carried out under aerobic conditions, for shaking or under aeration and agitation. The cultivation temperature is generally within the range of 20 to 50 °C. The pH of the culture medium is desirably maintained at neutrality or slight alkalinity. The cultivation period is usually about 5 hours to about 3 days.

By using the microorganism cells obtained by the above cultivation method or the treated product thereof, indole or 5-hydroxyindole, fumaric acid or its salts and the ammonium ion are reacted in accordance with this invention. When indole is used, L-tryptophan is formed in a high yield. The use of 5-hydroxyindole gives L-5-hydroxytryptophan in a high yield.

The L-tryptophan or L-5-hydroxytryptophan formed in the reaction mixture may be isolated and purified by methods known per se, for example, by adsorption and desorption using ion exchange resins.

The following Referential Examples and Examples illustrate the present invention more specifically.

In the following examples, L-tryptophan and L-5-hydroxytryptophan were identified by the following procedure.

The formation of L-tryptophan was determined by a bioassay using Leuconostoc mesenteroides (ATCC 8042) which is a lactic acid bacterium. Its amount was determined by the same method and high-performance liquid chromatography.

The formation of L-5-hydroxytryptophan was determined by paper chromatography, developing the paper with a 4:1:2 mixture of butanol, acetic acid and water, and performing a color reaction on the chromatogram using the Ehrlich reagent. The amount of L-5-hydroxytryptophan was determined by using high-performance liquid chromatography.

REFERENTIAL EXAMPLE 1

Preparation of Brevibacterium flavum cells

One hundred milliliters of a medium having the composition shown in Table 1 below was poured into each of two 500 ml Erlenmeyer flasks, and heat-sterilized at 120 °C for 15 minutes. Ethanol (2 % by volume) was aseptically added to each of the media, and one platinum loopful of Brevibacterium flavum MJ-233 (FERM BP-1497) or Brevibacterium flavum MJ-233-AB-41 (FERM BP-1498) was inoculated in the medium, and cultivated at 30 °C for 24 hours.

Twenty milliliters of these two sets of the culture broth was inoculated separately in 1 liter of a culture medium having the composition shown in Table 2 in a 2-liter jar fermentor. The medium was stirred at 33 °C and a pH of 7.6 with 1 vvm of air passed through the fermentor, and ethanol was intermittently added so that the concentration of ethanol was maintained at 1.0 to 1.5 % by volume. After cultivation for 30 hours, the culture broth was centrifuged (6,000 rpm, 15 minutes), and the resulting cells were used as test cells.

Table 1

| | |
|---|---|
| Urea | 4.0 g |
| Ammonium sulfate | 14.9 g |
| $KH_2PO_4$ | 0.5 g |
| $K_2HPO_4$ | 0.5 g |
| $MgSO_4.7H_2O$ | 0.5 g |
| $FeSO_4.7H_2O$ | 6.0 mg |
| $MnSO_4.4 - 6H_2O$ | 6.0 mg |
| Yeast extract | 1.0 g |
| Casamino acids | 1.0 g |
| Biotin | 200 $\mu$g |
| Thiamine hydrochloride | 100 $\mu$g |
| Distilled water | 1000 ml |

Table 2

| | |
|---|---|
| Ammonium sulfate | 23.0 g |
| $KH_2PO_4$ | 0.5 g |
| $K_2HPO_4$ | 0.5 g |
| $MgSO_4.7H_2O$ | 0.5 g |
| $FeSO_4.7H_2O$ | 20 mg |
| $MnSO_4.4 - 6H_2O$ | 20 mg |
| Yeast extract | 3 g |
| Casamino acids | 3 g |
| Biotin | 200 $\mu$g |
| Thiamine hydrochloride | 100 $\mu$g |
| Distilled water | 1000 ml |

REFERENTIAL EXAMPLE 2

Preparation of Escherichia coli cells

Fifty milliliters of a medium having the composition shown in Table 3 below was put in a 500 ml Erlenmeyer flask, and sterilized at 120°C for 15 minutes. Escherichia coli K-12 strain [ATCC 27325, YK3002 FERM BP-1733 and YK3003 FERM BP-1734) was inoculated and cultivated at 37 °C for 1 day with shaking. Twenty milliliters of the culture was inoculated in 100 ml of an L broth containing L-tryptophan in a concentration of 200 $\mu$g/ml prepared and sterilized in the same way as above, and cultivated at 37 °C for 8 hours. The culture broth was centrifuged (6000 rpm, 15 minutes, 4 °C) to harvest the cells.

Table 3

| L broth | |
|---|---|
| Trypton | 10 g |
| Yeast extract | 5 g |
| NaCl | 5 g |
| Glucose | 1 g |
| Distilled water | 1 liter |
| (pH 7.2) | |

REFERENTIAL EXAMPLE 3

Preparation of Proteus morganii cells

Fifty milliliters of a medium having the composition shown in Table 4 was put in a 500 ml Erlenmeyer flask, and sterilized at 120 °C for 15 minutes. Proteus morganii (IFO-3838) was inoculated in the medium and cultivated at 37 °C for 24 hours. Twenty milliliters of the culture broth was inoculated in 1000 ml of a medium having the composition shown in Table 4 containing L-tryptophan in a concentration of 200 $\mu$g/ml sterilized in the same way as above, and cultivated at 37 °C for 8 hours with shaking. The culture broth was centrifuged (6000 rpm, 15 minutes, 4 °C) to harvest the cells.

Table 4

| | |
|---|---|
| $KH_2PO_4$ | 0.5 g/liter |
| $K_2HPO_4$ | 0.5 g/liter |
| $MgSO_4.7H_2O$ | 0.5 g/liter |
| $FeSO_4.7H_2O$ | 6 ppm |
| $MnSO_4.nH_2O$ | 6 ppm |
| Yeast extract | 10 g/liter |
| Casamino acids | 5 g/liter |
| (pH 7.5) | |

REFERENTIAL EXAMPLE 4

Preparation of Erwinia herbicola cells

Erwinia herbicola (ATCC 21433) was cultivated at 37 °C for 8 hours with shaking in the same medium and by the same operation as described in Referential Example 3. The culture broth was centrifuged (6000 rpm, 15 minutes, 4 °C) to harvest the cells.

REFERENTIAL EXAMPLE 5

Production of NADH oxidase

Leuconostoc mesenteroides (ATCC 9135) was cultivated by the method of Kawai et al. described in The Journal of General and Applied Microbiology, vol. 17, pages 51-52, 1971. The resulting NADH oxidase was purified to obtain NADH oxidase having a specific activity of about 160 (units/mg) (1 unit is the amount of the enzyme which can regenerate 1 micromole of a coenzyme per minute).

REFERENTIAL EXAMPLE 6

Preparation of Esherichia coli cells

Fifty milliliters of a medium having the composition shown in Table 5 was put in a 500 ml Erlenmeyer flask, and sterilized at 120 °C for 15 minutes, and a separately sterilized aqueous solution of glucose or L-malic acid (adjusted to pH 7.0 with 5N NaOH) was added to the sterilized medium so as to provide a concentration of 1 % (wt/vol). One loopful of Escherichia coli K-12 strain [YK3002 (FERM BP-1733), YK3003 (FERM BP-1734) or YK3004 (FERM BP-1735)] was inoculated in the resulting medium, and cultivated at 37 °C for 1 day with shaking. Twenty milliliters of the culture broth was inoculated in 1000 ml of a medium having the composition shown in Table 5 and containing 200 $\mu$g/ml of L-tryptophan and 1 % (wt/vol) of glucose or L-malic acid, prepared and sterilized as above, and cultivated at 37 °C for 8 hours with shaking. The culture broth was centrifuged (6000 rpm, 15 minutes, 4 °C) to harvest the cells.

Table 5

| Peptone | 1 g |
|---|---|
| Yeast extract | 1 g |
| Ammonium sulfate | 3 g |
| $KH_2PO_4$ | 1.6 g |
| $K_2HPO_4$ | 5.5 g |
| $MgSO_4.7H_2O$ | 0.2 g |
| $FeSO_4.7H_2O$ | 50 mg |
| Distilled water | 1000 ml |
| pH 7.2 | |

REFERENTIAL EXAMPLE 7

One gram of the Escherichia coli cells K-12 strain [YK3002 (FERM BP-1733), YK3003 (FERM BP-1734) or YK3004 (FERM BP-1735)] cultivated by using glucose or L-malic acid as a main carbon source as shown in Referential Example 6 were suspended in 2 ml of a 50 mM phosphate buffer (pH 7.0), disrupted by ultrasonic oscillation and centrifuged (12000 rpm, 40 minutes, 4 °C) to obtain a supernatant. The tryptophanase and pyruvic acid-malic acid carboxylase activities of this fraction were measured.

The tryptophanase activity was measured as follows:

0.1 ml of the above supernatant diluted properly was added to 0.9 ml of a reaction solution containing 100 $\mu$mole of phosphate buffer (pH 8.0), 5 $\mu$mole of L-tryptophan and 0.04 $\mu$mole of pyridoxalphosphate and reacted at 37 °C for 20 minutes. Then, in accordance with a usual method [O. H. Smith and C. Yanofsky, "Methods in Enzymology", Academic Press, New York, vol. 5, pp. 794-806 (1962)], the amount of the resulting indole was determined, and from it, the tryptophanase activity was calculated.

The pyruvic acid-malic acid carboxylase activity was determined by measuring the increase of absorbance by NADH in accordance with the method of Murai et al. described in The Journal of Biochemistry, vol. 71, pages 1015-1028 (1972).

The measured enzyme activities for glucose and L-malic acid as a carbon source are shown in Table 6. The indicated activities are relative values when the activity obtained by using glucose as a main carbon source is taken as 100.

Table 6

| Activity / Strain | Tryptophanase activity | | Pyruvic acid-malic acid carboxylase activity | |
|---|---|---|---|---|
| | Glucose | L-malic acid | Glucose | L-malic acid |
| YK 3002 (FERM BP-1733) | 100 | 99 | 100 | 151 |
| YK 3003 (FERM BP-1734) | 100 | 101 | 100 | 149 |
| YK 3004 (FERM BP-1735) | 100 | 100 | 100 | 149 |

EXAMPLE 1

Fifty milliliters of a reaction solution having the composition shown in Table 7 was put in an Erlenmeyer flask. The cells obtained by centrifuging (6000 rpm, 15 minutes, 4 °C) 40 ml of the culture broth of Brevibacterium flavum MJ-233 (FERM BP-1497) prepared in Referential Example 1, the cells obtained by centrifuging (6000 rpm, 15 minutes, 4 °C) 40 ml of the culture broth of Escherichia coli K-12 (ATCC 27325) prepared in Referential Example 2, and 2 ml of the NADH oxidase shown in Referential Example 5 were added to the flask. The flask was shaken at 37 °C for 24 hours. After the reaction, the cells were removed by centrifugal separation (4000 rpm, 15 minutes, room temperature). The L-tryptophan in the supernatant was quantitatively determined, and it was found that 65 mg/liter of L-tryptophan was formed.

The cells recovered from 40 ml of the culture broth of Brevibacterium flavum MJ-233 (FERM BP-1497) prepared in Referential Example 1 and the cells recovered from 40 ml of the culture broth of Escherichia coli K-12 (ATCC 27325) were added to the above reaction solution and reacted at 37 °C for 24 hours with shaking. The amount of L-tryptophan formed was 21 mg/liter.

Table 7

| Indole | 20 mM |
| Sodium fumarate | 50 mM |
| Ammonium chloride | 300 mM |
| Nicotinamideadenine dinucleotide (NAD$^+$) | 0.1 mM |
| Phosphate buffer (pH 8.0) | 100 mM |

EXAMPLE 2

The cells obtained from 40 ml of the culture broth of Brevibacterium flavum MJ-233-AB-41 (FERM BP-1498) prepared in Referential Example 1, the cells obtained from 40 ml of the culture broth of Escherichia coli K-12 YK3002 (FERM BP-1733) prepared in Referential Example 2, and 2 ml of the NADH oxidase solution prepared in Referential Example 5 were added to 50 ml of the same reaction solution as used in Example 1, and the reaction solution was reacted at 37 °C for 15 hours. After the reaction, the amount of L-tryptophan formed in the supernatant was determined to be 70 mg/liter.

Forty milliliters of the supernatant was passed through a column of an ammonia-type strongly acidic ion exchange resin (Diaion SK-1B produced by Mitsubishi Chemical Co., Ltd.) to cause adsorption of L-tryptophan. The column was then eluted with an alkaline solution. The eluate was concentrated to precipitate crude crystals of L-tryptophan. The crude crystals were washed with acetone and dried to give 2.0 mg of crystals of L-tryptophan.

When only the cells obtained from 40 ml of the culture broth of Brevibacterium flavum MJ-233-AB-41 (FERM BP-1498) and the cells obtained from 40 ml of the culture broth of Escherichia coli K-12 YK3002 (FERM BP-1733) were used and the reaction was carried out in the same way as in Example 1, the amount of L-tryptophan formed in the supernatant after the reaction was 25 mg/liter.

EXAMPLE 3

The cells obtained from 40 ml of the culture broth of Brevibacterium flavum MJ-233-AB-41 (FERM BP-1498) prepared in Referential Example 1, the cells obtained from 40 ml of the culture broth of Escherichia coli K-12 YK3003 (FERM BP-1734) prepared in Referential Example 2, and 2 ml of the NADH oxidase solution prepared in Referential Example 5 were added to 50 ml of the same reaction solution as used in Example 1, and the reaction solution was reacted at 37 °C for 15 hours. After the reaction, the amount of L-tryptophan formed in the supernatant was determined to be 66 mg/liter.

Forty milliliters of the supernatant was passed through a column of an ammonia-type strongly acidic ion exchange resin (Diaion SK-1B produced by Mitsubishi Chemical Co., Ltd.) to cause adsorption of L-tryptophan. The column was then eluted with an alkaline solution. The eluate was concentrated to precipitate crude crystals of L-tryptophan. The crude crystals were washed with acetone and dried to give 1.9 mg of crystals of L-tryptophan.

When only the cells obtained from 40 ml of the culture broth of Brevibacterium flavum MJ-233-AB-41 (FERM BP-1498) and the cells obtained from 40 ml of the culture broth of Escherichia coli K-12 YK3003 (FERM BP-1734) were used and the reaction was carried out in the same way as in Example 1, the amount of L-tryptophan formed in the supernatant after the reaction was 24 mg/liter.

EXAMPLE 4

The cells obtained from 40 ml of the culture broth of Brevibacterium flavum MJ-233-AB-41 (FERM BP-1498) prepared in Referential Example 1, the cells obtained from 80 ml of the culture broth of Proteus morganii (IFO-3848) prepared in Referential Example 3, and 2 ml of the NADH oxidase solution prepared in Referential Example 5 were added to 50 ml of the same reaction solution as used in Example 1, and the reaction solution was reacted at 37 °C for 24 hours with shaking. The amount of L-tryptophan formed in the supernatant was determined to be 62 mg/liter.

By the same operation as in Example 2, L-tryptophan was recovered from 40 ml of the reaction supernatant. There were obtained 1.7 mg of crystals of L-tryptophan.

When only the cells obtained from 40 ml of the culture broth of Brevibacterium flavum MJ-233-AB-41 (FERM BP-1498) and 80 ml of the culture broth of Proteus morganii (IFO-3848) were used and the reaction was carried out in the same way as in Example 1, the amount of L-tryptophan formed in the supernatant after the reaction was 22 mg/liter.

EXAMPLE 5

The same reaction as in Example 4 was carried out except that the Erwinia herbicola (ATCC 21433) cells prepared in Referential Example 4 were used instead of the Proteus morganii cells. The amount of L-tryptophan formed in the supernatant after the reaction was determined to be 64 mg/liter.

When only the cells obtained from 40 ml of the culture broth of Brevibacterium flavum MJ-233-AB-41 (FERM BP-1498) and 80 ml of the culture broth of Erwinia herbicola (ATCC 21433) were used and subjected to the same reaction as in Example 1, the amount of L-tryptophan formed after the reaction was 20 mg/liter.

EXAMPLE 6

Fifty milliliters of a reaction solution having the composition shown in Table 8 was put in a 500 ml Erlenmeyer flask, and then reacted by the same operation as in Example 2. After the reaction, the amount of L-5-hydroxytryptophan in the supernatant was determined to be 63 mg/liter.

Forty milliliters of the supernatant was passed through a column of an ammonia-type strongly acidic ion exchange resin (Diaion SK-1B produced by Mitsubishi Chemical Co., Ltd.) to cause adsorption of L-5-hydroxytryptophan. The column was then eluted with an alkaline solution. The eluate was concentrated to precipitate crude crystals of L-5-hydroxytryptophan. The crude crystals were washed with acetone and dried to give 1.8 mg of crystals of L-5-hydroxytryptophan.

When only the cells obtained from 40 ml of the culture broth of Brevibacterium flavum MJ-233-AB-41 (FERM BP-1498) and the cells obtained from 40 ml of the culture broth of Escherichia coli K-12 YK3002 (FERM BP-1733) were used and the reaction was carried out in the same way as in Example 1, the amount of L-5-hydroxytryptophan formed in the supernatant after the reaction was 19 mg/liter.

Table 8

| 5-Hydroxyindole | 20 mM |
| Sodium fumarate | 50 mM |
| Ammonium chloride | 300 mM |
| Nicotinamideadenine dinucleotide (NAD$^+$) | 0.1 mM |
| Phosphate buffer (pH 8.0) | 100 mM |

EXAMPLE 7

The same reaction as in Example 6 was carried out except that Escherichia coli K-12 YK3003 (FERM BP-1734) was used instead of Escherichia coli K-12 YK3002 (FERM BP-1733). The amount of L-5-hydroxytryptophan in the supernatant after the reaction was determined to be 64 mg/liter.

10

Forty milliliters of the supernatant was passed through a column of an ammonia-type strongly acidic ion exchange resin (Diaion SK-1B produced by Mitsubishi Chemical Co., Ltd.) to cause adsoprtion of L-5-hydroxytryptophan. The column was then eluted with an alkaline solution. The eluate was concentrated to precipitate crude crystals of L-5-hydroxytryptophan. The crude crystals were washed with acetone and dried to give 1.9 mg of crystals of L-5-hydroxytryptophan.

When only the cells obtained from 40 ml of the culture broth of Brevibacterium flavum MJ-233-AB-41 (FERM BP-1498) and the cells obtained from 40 ml of the culture broth of Escherichia coli K-12 YK3003 (FERM BP-1734) were used and the reaction was carried out in the way as in Example 1, the amount of L-5-hydroxytryptophan formed in the supernatant after the reaction was 20 mg/liter.

## EXAMPLE 8

Fifty milliliters of a reaction solution having the composition shown in Table 8 was put in a 500 ml Erlenmeyer flask, and then reacted by the same operation as in Example 4. After the reaction, the amount of L-5-hydroxytryptophan in the supernatant was determined to be 66 mg/liter.

Forty milliliters of the supernatant was passed through a column of an ammonia-type strongly acidic ion exchange resin (Diaion SK-1B produced by Mitsubishi Chemical Co., Ltd.) to cause adsorption of L-5-hydroxytryptophan. The column was then eluted with an alkaline solution. The eluate was concentrated to precipitate crude crystals of L-5-hydroxytryptophan. The crude crystals were washed with acetone and dried to give 2.0 mg of crystals of L-5-hydroxytryptophan.

When only the cells obtained from 40 ml of the culture broth of Brevibacterium flavum MJ-233-AB-41 (FERM BP-1498) and the cells obtained from 80 ml of the culture broth of Proteus morganii (IFO-3848) were used and the reaction was carried out in the same way as in Example 6, the amount of L-5-hydroxytryptophan formed in the supernatant after the reaction was 21 mg/liter.

## EXAMPLE 9

Sodium sulfite (0.2 %, wt/vol) was added to 50 ml of a reaction solution having the composition shown in Table 9, and the mixture was put in a 500 ml Erlenmeyer flask. Then, the cells obtained by centrifuging (6000 rpm, 15 minutes, 4 °C) 40 ml of the culture broth of Brevibacterium flavum MJ-233 (FERM BP-1497) prepared in Referential Example 1, the cells obtained by centrifuging (6000 rpm, 15 minutes, 4 °C) 40 ml of the culture broth of Escherichia coli K-12 (ATCC 27325) prepared in Referential Example 2 and 2 ml of the NADH oxidase solution shown in Referentaial Example 5 were added to the reaction solution and the reaction solution was reacted at 37 °C for 24 hours with shaking. After the reaction, the reaction mixture was centrifuged (4000 rpm, 15 minutes, room temperature) to remove the cells. The amount of L-tryptophan in the supernatant was determined to be 118 mg/liter.

When only the cells recovered from 40 ml of the culture broth of Brevibacterium flavum MJ-233 (FERM BP-1497) prepared in Referential Example 1 and the cells recovered from 40 ml of the culture broth of Escherichia coli K-12 (ATCC 27325) were added to the above reaction solution and the reaction was carried out at 37 °C for 24 hours with shaking, the amount of L-tryptophan formed was 21 mg/liter.

When the above reaction was carried out without adding sodium sulfite to the reaction solution, the amount of L-tryptophan formed was 65 mg/liter.

Table 9

| | |
|---|---|
| Indole | 20 mM |
| Sodium fumarate | 50 mM |
| Ammonium chloride | 300 mM |
| NAD$^+$ | 0.1 mM |
| Phosphate buffer (pH 8.0) | 100 mM |

## EXAMPLE 10

Sodium sulfite (0.2 %, wt/vol) was added to 50 ml of the same reaction solution as used in Example 9. The cells obtained from 40 ml of the culture broth of Brevibacterium flavum MJ-233-AB-41 (FERM BP-1498) prepared in Referential Example 1, the cells from 40 ml of the culture broth of Escherichia coli K-12 YK3002 (FERM BP-1733) prepared in Referential Example 2 and 2 ml of the NADH oxidase solution prepared in Referential Example 5 were added to the mixture, and the reaction solution was reacted at 37 °C for 15 hours with shaking. After the reaction, the amount of L-tryptophan formed in the supernatant was determined to be 128 mg/liter.

Forty milliliters of the reaction supernatant was passed through a column of an ammonia-type strongly acidic ion exchange resin (Diaion SK-1B, produced by Mitsubishi Chemical Co., Ltd.) to cause adsorption of L-tryptophan. The column was eluted with an alkaline solution. The eluate was concentrated to precipitate crude crystals of L-tryptophan. The crude crystals were washed with acetone and dried to give 3.6 mg of crystals of L-tryptophan.

When only the cells obtained from 40 ml of the culture broth of Brevibacterium flavum MJ-233-AB-41 (FERM BP-1498) and the cells obtained from 40 ml of the culture broth of Escherichia coli K-12 (FERM BP-1733) were used and the reaction was carried out in the same way as in Example 9, the amount of L-tryptophan formed in the supernatant was 25 mg/liter.

When the above reaction was carried out without adding sodium sulfite to the reaction solution, the amount of L-tryptophan formed by the same reaction as above was 70 mg/liter.

EXAMPLE 11

Sodium sulfite (0.2 %, wt/vol) was added to 50 ml of the same reaction solution as used in Example 9. The cells obtained from 40 ml of the culture broth of Brevibacterium flavum MJ-233-AB-41 (FERM BP-1498) prepared in Referential Example 1, the cells from 40 ml of the culture broth of Escherichia coli K-12 YK3003 (FERM BP-1734) prepared in Referential Example 2 and 2 ml of the NADH oxidase solution prepared in Referential Example 5 were added to the mixture, and the reaction was carried out at 37 °C for 15 hours with shaking. After the reaction, the amount of L-tryptophan formed in the supernatant was determined to be 118 mg/liter.

Forty milliliters of the reaction supernatant was passed through a column of an ammonia-type strongly acidic ion exchange resin (Diaion SK-1B, produced by Mitsubishi Chemical Co., Ltd.) to cause adsorption of L-tryptophan. The column was eluted with an alkaline solution. The eluate was concentrated to precipitate crude crystals of L-tryptophan. The crude crystals were washed with acetone and dried to give 3.1 mg of crystals of L-tryptophan.

When only the cells obtained from 40 ml of the culture broth of Brevibacterium flavum MJ-233-AB-41 (FERM BP-1498) and the cells obtained from 40 ml of the culture broth of Escherichia coli K-12 YK3003 (FERM BP-1734) were used and the reaction was carried out in the same way as in Example 9, the amount of L-tryptophan formed in the supernatant was 24 mg/liter.

When the above reaction was carried out without adding sodium sulfite to the reaction solution, the amount of L-tryptophan formed by the same reaction as above was 66 mg/liter.

EXAMPLE 12

Sodium sulfite (0.2 %, wt/vol) was added to 50 ml of the same reaction solution as used in Example 9. The cells obtained from 40 ml of the culture broth of Brevibacterium flavum MJ-233-AB-41 (FERM BP-1498) prepared in Referential Example 1, the cells from 80 ml of the culture broth of Proteus morganii (IFO-3848) prepared in Referential Example 3 and 2 ml of the NADH oxidase solution prepared in Referential Example 5 were added to the mixture, and reacted at 37 °C for 24 hours with shaking. After the reaction, the amount of L-tryptophan formed in the supernatant was determined to be 109 mg/liter.

By the same operation as in Example 10, 3.0 mg of crystals of L-tryptophan were recovered from the reaction supernatant.

When only the cells obtained from 40 ml of the culture broth of Brevibacterium flavum MJ-233-AB-41 (FERM BP-1498) and the cells obtained from 80 ml of the culture broth of Proteus morganii (IFO-3848) and the reaction was carried out in the way as in Example 9, the amount of L-tryptophan formed in the supernatant was 22 mg/liter.

When the above reaction was carried out without adding sodium sulfite to the reaction solution, the amount of L-tryptophan formed by the same reaction as above was 62 mg/liter.

EXAMPLE 13

The same reaction as in Example 12 was carried out except that the Erwinia herbicola (ATCC 21433) cells prepared in Referential Example 4 were used instead of the Proteus morganii (IFO-3848) cells. The amount of L-tryptophan formed in the supernatant after the reaction was determined to be 113 mg/liter.

When only the cells obtained from 40 ml of the culture broth of Brevibacterium flavum MJ-233-AB-41 (FERM BP-1498) and 80 ml of the culture broth of Erwinia hervicola (ATCC-21433) were used and the reaction was carried out in the same way as in Example 9, the amount of L-tryptophan formed after the reaction was 20 mg/liter.

When the above reaction was carried out without adding sodium sulfite to the reaction solution, the amount of L-tryptophan formed was 64 mg/liter.

EXAMPLE 14

A reaction solution having the composition shown in Table 10 was put in a 500 ml Erlenmeyer flask. The cells obtained from 40 ml of the culture broth of Brevibacterium fluvum MJ-233 (FERM BP-1497) prepared in Referential Example 1, 2 ml of the NADH oxidase solution shown in Referential Example 5, and the cells obtained from 40 ml of the culture broth of Escherichia coli K-12 YK-3002 (FERM BP-1733) prepared in Referential Example 6 using L-malic acid as a main carbon source were added to the reaction solution, and the reaction was carried out at 37 °C for 24 hours. After the reaction, the reaction mixture was centrifuged to remove the cells. The amount of L-tryptophan in the supernatant was determined to be 98 mg/liter.

Table 10

| Indole | 20 mM |
| Sodium fumarate | 50 mM |
| Ammonium chloride | 300 mM |
| $NAD^+$ | 0.1 mM |
| $MnCl_2$ | 2 mM |
| Phosphate buffer (pH 8.0) | 100 mM |

Forty milliliters of the supernatant was passed through a column of an ammonia-type strongly acidic ion exchange resin (Diaion SK-1B produced by Mitsubishi Chemical Co., Ltd.) to cause adsorption of L-tryptophan. The column was then eluted with an alkaline solution. The eluate was concentrated to precipitate crude crystals of L-tryptophan. The crude crystals were washed with acetone and dried to give 2.9 mg of crystals of L-tryptophan.

EXAMPLE 15

A solution having the composition shown in Table 10 in Example 14 was put in a 500 ml Erlenmeyer flask. The cells obtained from 40 ml of the culture broth of Brevibacterium flavum MJ-233-AB-41 (FERM BP-1498) prepared in Referential Example 1, 2 ml of the NADH oxidase solution shown in Referential Example 5, and the cells obtained from 40 ml of the culture broth of Escherichia coli K-12 YK-3002 (FERM BP-1733) prepared in Referential Example 6 using L-malic acid as a main carbon source were added to the reaction solution, and the reaction was carried out at 37 °C for 15 hours with shaking. After the reaction, the reaction mixture was centrifuged to remove the cells. The amount of L-tryptophan in the supernatant was found to be 104 mg/liter.

Forty milliliters of the reaction supernatant was passed through a column of an ammonia-type strongly acidic ion-exchange resin (Diaion SK-1B produced by Mitsubishi Chemical Co., Ltd.) to cause adsorption of L-tryptophan. The column was eluted with an alkaline solution. The eluate was concentrated to precipitate crude crystals of L-tryptophan. The crude crystals were washed with acetone, and dried to give 3.0 mg of crystals of L-tryptophan.

EXAMPLE 16

A solution having the composition shown in Table 10 in Example 14 was put in a 500 ml Erlenmeyer flask. The cells obtained from 40 ml of the culture broth of Brevibacterium flavum MJ-233-AB-41 (FERM BP-1498) prepared in Referential Example 1, 2 ml of the NADH oxidase solution shown in Referential Example 5, and the cells obtained from 40 ml of the culture broth of Escherichia coli K-12 YK-3003 (FERM BP-1734) prepared in Referential Example 6 using L-malic acid as a main carbon source were added to the

13

reaction solution, and the reaction was carried out at 37 °C for 15 hours with shaking. After the reaction, the reaction mixture was centrifuged to remove the cells. The amount of L-tryptophan in the supernatant was found to be 99 mg/liter.

Forty milliliters of the reaction supernatant was passed through a column of an ammonia-type strongly acidic ion-exchange resin (Diaion SK-1B produced by Mitsubishi Chemical Co., Ltd.) to cause adsorption of L-tryptophan. The column was eluted with an alkaline solution. The eluate was concentrated to precipitate crude crystals of L-tryptophan. The crude crystals were washed with acetone, and dried to give 3.0 mg of crystals of L-tryptophan.

EXAMPLE 17

Fifty milliliters of a reaction solution having the composition shown in Table 11 was put in a 500 ml Erlenmeyer flask. The cells obtained by centrifuging (6000 rpm, 15 minutes, 4 °C) 40 ml of the culture broth of Brevibacterium flavum MJ-233 (FERM BP-1497) prepared in Referential Example 1, cells obtained by centrifuging (6000 rpm, 15 minutes, 4 °C) 40 ml of the culture broth of Escherichia coli K-12 (ATCC 27325) prepared in Referential Example 2, and 2 ml of the NADH oxidase solution shown in Referential Example 5 were added to the reaction solution, and reacted at 37 °C for 24 hours with shaking. While the concentration of indole in the reaction solution was monitored, indole was consecutively added so that the indole concentration in the reaction solution did not exceed 4 mM. The reaction was carried out until the final total concentration of the indole added reached 10 mM. After the reaction, the cells were removed by centrifugation (4000 rpm, 15 minutes, room temperature). The amount of L-tryptophan in the supernatant was found to be 1240 mg/liter.

Table 11

| Indole | 4 mM (initial concentration) |
|---|---|
| Sodium fumarate | 50 mM |
| Ammonium chloride | 300 mM |
| NAD$^+$ | 0.1 mM |
| Phosphate buffer (pH 8.0) | 100 mM |

EXAMPLE 18

The cells obtained from 40 ml of the culture broth of Brevibacterium flavum MJ-233-AB-41 (FERM BP-1498) prepared in Referential Example 1, the cells obtained from 40 ml of the culture broth of Escherichia coli K-12 YK3002 (FERM BP-1733) prepared in Referential Example 2, and 2 ml of the NADH oxidase prepared in Referential Example 5 were added to 50 ml of the same reaction solution as used in Example 17, and the reaction was carried out at 37 °C for 15 hours with shaking while adding indole in the same way as in Example 17. After the reaction, the amount of L-tryptophan in the supernatant was determined to be 1840 mg/liter.

Forty milliliters of the reaction supernatant was passed through a column of an ammonia-type strongly acidic ion-exchange resin (Diaion SK-1B produced by Mitsubishi Chemical Co., Ltd.) to cause adsorption of L-tryptophan. The column was eluted with an alkaline solution. The eluate was concentrated to precipitate crude crystals of L-tryptophan. The crude crystals were washed with acetone, and dried to give 48 mg of crystals of L-tryptophan.

EXAMPLE 19

The cells obtained from 40 ml of the culture broth of Brevibacterium flavum MJ-233-AB-41 (FERM BP-1498) prepared in Referential Example 1, the cells obtained from 40 ml of the culture broth of Escherichia coli K-12 YK3003 (FERM BP-1734) prepare in Referential Example 2, and 2 ml of the NADH oxidase solution prepared in Referential Example 5 were added to 50 ml of the same reaction solution as used in Example 17, and the reaction was carried out at 37 °C for 15 hours with shaking while adding indole in the same way as in Example 17. After the reaction, the amount of L-tryptophan in the supernatant was determined to be 1830 mg/liter.

EP 0 295 622 B1

Forty milliliters of the reaction supernatant was passed through a column of an ammonia-type strongly acidic ion-exchange resin (Diaion SK-1B produced by Mitsubishi Chemical Co., Ltd.) to cause adsorption of L-tryptophan. The column was eluted with an alkaline solution. The eluate was concentrated to precipitate crude crystals of L-tryptophan. The crude crystals were washed with acetone, and dried to give 47 mg of crystals of L-tryptophan.

**Claims**

1. A process for producing L-tryptophan optionally substituted at the 5-position by a hydroxyl group, which comprises
    (i) enzymatically reacting fumaric acid or its salt, an ammonium ion and indole optionally substituted at the 5-position by a hydroxyl group in the presence of
        (A) a microorganism cell of Brevibacterium flavum MJ-233 (FERM BP-1497) or Brevibacterium flavum MJ-233-AB-41 (FERM BP-1498), or a treated product thereof,
        (B) a microorganism cell of Escherichia coli K-12 YK 3002 (FERM BP-1733), Escherichia coli K-12 YK 3003 (FERM BP-1734) or Escherichia coli K-12 YK 3004 (FERM BP-1735), or a treated product thereof and
        (C) NADH oxidase, and
    (ii) recovering L-tryptophan optionally substituted at the 5-position by a hydroxyl group from the reaction mixture.

2. The process of claim 1 wherein the microorganism cell of Escherichia coli K-12 YK 3002 (FERM BP-1733), Escherichia coli K-12 YK 3003 (FERM BP-1734) or Escherichia coli K-12 YK 3004 (FERM BP-1735) is a microorganism cell obtained by culturing said microorganisms in a medium containing malic acid or its salt as a main carbon source, or a treated product thereof.

3. The process of claim 1 wherein the NADH oxidase (C) is FROM a microorganism cell of Leuconostoc mesenteroides (ATCC 9135) or Bacillus cereus (ATCC 4342), or a treated product thereof.

4. The process of claim 1 wherein the concentrations of fumaric acid or its salt and the ammonia ion in the reaction system are 0.1 to 20 % (wt/vol).

5. The process of claim 1 wherein the concentration of the indole optionally substituted by a hydroxyl group at the 5-position in the reaction system is 0.1 to 20 % (wt/vol).

6. The process of claim 1 wherein the indole optionally substituted at the 5-position by a hydroxyl is added continuously or intermittently under controlled conditions such that the average concentration of the indole does not exceed 4 mM.

7. The process of claim 1 wherein the reaction is carried out in the presence of a reducing agent.

8. The process of claim 7 wherein the reducing agent is selected from dithiothreitol, dithioerythritol, 2-mercaptoethanol, L-cysteine, L-ascorbic acid and sodium sulfite.

9. The process of claim 7 wherein the concentration of the reducing agent is 0.0001 to 10 % (wt/vol).

10. The process of claim 1 wherein oxidized-type or reducedtype nicotinamideadenine dinucleotide ($NAD^+$ or NADH), and/or a metal ion is added to the reaction system.

11. The process of claim 10 wherein the concentration of $NAD^+$ or NADH in the reaction system is 0.005 to 20 mM.

12. The process of claim 10 wherein the concentration of the metal ion in the reaction system is 0.1 to 20 mM.

15

**Patentansprüche**

1. Verfahren zur Herstellung von L-Tryptophan, welches gegebenenfalls in 5-Position durch eine Hydroxylgruppe substituiert ist, dadurch **gekennzeichnet,** daß man

   (i) enzymatisch Fumarsäure oder ihr Salz, ein Ammoniumion und Indol, das gegebenenfalls in der 5-Position durch eine Hydroxylgruppe substituiert ist, in Gegenwart von

   (A) einer Mikroorganismenzelle von Brevibacterium flavum MJ-233 (FERM BP-1497) oder Brevibacterium flavum MJ-233-AB-41 (FERM BP-1498), oder einem behandelten Produkt davon,

   (B) einer Mikroorganismenzelle von Escherichia coli K-12 YK 3002 (FERM BP-1733), Escherichia coli K-12 YK 3003 (FERM BP-1734) oder Escherichia coli K-12 YK 3004 (FERM BP-1735), oder einem behandelten Produkt davon und

   (C) NADH-Oxidase

   umsetzt, und

   (ii) L-Tryptophan, das gegebenenfalls in der 5-Position durch eine Hydroxylgruppe substituiert ist, aus dem Reaktionsgemisch isoliert.

2. Verfahren nach Anspruch 1, dadurch **gekennzeichnet,** daß die Mikroorganismenzelle von Escherichia coli K-12 YK 3002 (FERM BP-1733), Escherichia coli K-12 YK 3003 (FERM BP-1734) oder Escherichia coli K-12 YK 3004 (FERM BP-1735) eine Mikroorganismenzelle ist, die durch Züchten der Mikroorganismen in einem Medium, das Äpfelsaure oder ein Salz davon als Hauptkohlenstoffquelle enthält, oder ein behandeltes Produkt davon ist.

3. Verfahren nach Anspruch 1, dadurch **gekennzeichnet,** daß die NADH-Oxidase (C) aus einer Mikroorganismenzelle von Leuconostoc mesenteroides (ATCC 9135) oder Bacillus cereus (ATCC 4342), oder einem behandelten Produkt davon stammt.

4. Verfahren nach Anspruch 1, dadurch **gekennzeichnet,** daß die Konzentrationen an Fumarsäure oder einem Salz davon und des Ammoniumions in dem Reaktionssystem 0,1 bis 20 % (Gew./Vol.) betragen.

5. Verfahren nach Anspruch 1, dadurch **gekennzeichnet,** daß die Konzentration des Indols, das gegebenenfalls durch eine Hydroxylgruppe in der 5-Position substituiert ist, in dem Reaktionssystem 0,1 bis 20 % (Gew./Vol.) beträgt.

6. Verfahren nach Anspruch 1, dadurch **gekennzeichnet,** daß das Indol, das gegebenenfalls in der 5-Position durch eine Hydroxylgruppe substituiert ist, kontinuierlich oder mit Unterbrechungen unter kontrollierten Bedingungen zugesetzt wird, so daß die Durchschnittskonzentration des Indols 4 mM nicht überschreitet.

7. Verfahren nach Anspruch 1, dadurch **gekennzeichnet,** daß die Reaktion in Gegenwart eines Reduktionsmittels durchgeführt wird.

8. Verfahren nach Anspruch 7, dadurch **gekennzeichnet,** daß das Reduktionsmittel aus Dithiothreit, Dithioerythrit, 2-Mercaptoethanol, L-Cystein, L-Ascorbinsäure und Natriumsulfit ausgewählt wird.

9. Verfahren nach Anspruch 7, dadurch **gekennzeichnet,** daß die Konzentration des Reduktionsmittels 0,0001 bis 10 % (Gew./Vol.) beträgt.

10. Verfahren nach Anspruch 1, dadurch **gekennzeichnet,** daß Nicotinamidadenindinucleotid in oxidierter oder reduzierter Form (NAD$^+$ oder NADH) und/oder ein Metallion dem Reaktionssystem zugesetzt werden.

11. Verfahren nach Anspruch 10, dadurch **gekennzeichnet,** daß die Konzentration an NAD$^+$ oder NADH in dem Reaktionssystem 0,005 bis 20 mM beträgt.

12. Verfahren nach Anspruch 10, dadurch **gekennzeichnet,** daß die Konzentration des Metallions in dem Reaktionssystem 0,1 bis 20 mM beträgt.

**Revendications**

1. Un procédé pour produire du L-tryptophanne éventuellement substitué en position 5 par un groupe hydroxyle, qui comprend :

    (i) la réaction enzymatique de l'acide fumarique, ou de son sel, d'un ion ammonium et de l'indole éventuellement substitué en position 5 par un groupe hydroxyle, en présence :

        (A) d'une cellule du microorganisme Brevibacterium flavum MJ-233 (FERM BP-1497) ou Brevibacterium flavum MJ-233-AB-41 (FERM BP-1498), ou d'un produit de leur traitement,

        (B) une cellule du microorganisme Escherichia coli K-12 YK 3002 (FERM BP-1733), Escherichia coli K-12 YK 3003 (FERM BP-1734), ou Eschericha coli K-12 YK 3004 (FERM BP-1735) ou d'un produit de leur traitement,

        et (C) la NADH oxydase, et

    (ii) la récupération du L-tryptophanne, éventuellement substitué en position 5 par un groupe hydroxyle, que l'on extrait du mélange réactionnel.

2. Procédé selon la revendication 1, dans lequel la cellule de microorganisme Escherichia coli K-12 YK 3002 (FERM BP-1733), de Escherichia coli K-12 YK 3003 (FERM BP-1734) ou de Escherichia coli K-12 YK 3004 (FERM BP-1735) est une cellule du microorganisme obtenue par la culture desdits microorganismes dans un milieu contenant de l'acide malique ou son sel comme source principale de carbone, ou est un produit de traitement de ces cellules.

3. Procédé selon la revendication 1, dans lequel la NADH oxydase (C) provient d'une cellule de microorganisme de Leuconostoc mesenteroides (ATCC 9135) ou de Bacillus cereus (ATCC 4342), ou un produit de leur traitement.

4. Procédé selon la revendication 1, dans lequel la concentration de l'acide fumarique ou de son sel et celle de l'ion ammonium dans le système de réaction valent 0,1 à 20 % en poids/volume.

5. Procédé selon la revendication 1, dans lequel la concentration de l'indole, éventuellement substitué par un groupe hydroxyle en position 5, est de 0,1 à 20 % (en poids/volume) dans le système de réaction.

6. Procédé selon la revendication 1, dans lequel on ajoute de façon continue ou intermittente l'indole, éventuellement substitué en position 5 par un groupe hydroxyle, en opérant dans les conditions réglées telles que la concentration moyenne de l'indole n'excède pas 4 mM.

7. Procédé selon la revendication 1, dans lequel on effectue la réaction en présence d'un agent réducteur.

8. Procédé selon la revendication 7, dans lequel l'agent réducteur est choisi parmi le dithiotréitol, le dithioérythritol, le 2-mercaptoéthanol, la L-cystéine, l'acide L-ascorbique et le sulfite de sodium.

9. Procédé selon la revendication 7, dans lequel la concentration de l'agent réduceur est de 0,0001 à 10 % (en poids/volume).

10. Procédé selon la revendication 1, dans lequel on ajoute au système de réaction le dinucléotide nicotinamideadénine, de type oxydé ou de type réduit (NAD+ ou NADH), et/ou un ion de métal.

11. Procédé selon la revendication 10, dans lequel la concentration de NAD+ ou de NADH dans le système de réaction est de 0,0005 à 20 mM.

12. Procédé selon la revendication 10, dans lequel la concentration de l'ion métal dans le système de réaction va de 0,1 à 20 mM.